(19) Europäisches Patentamt
European Patent Office
Office européen des brevets

(11) Numéro de publication : **0 172 066**
**B1**

(12) **FASCICULE DE BREVET EUROPÉEN**

(45) Date de publication du fascicule du brevet : **30.09.87**

(21) Numéro de dépôt : **85401411.5**

(22) Date de dépôt : **11.07.85**

(51) Int. Cl.[4] : **C 07 C 69/65**, C 07 C 67/317, C 07 C 49/227, C 07 C 45/67, C 07 C147/14

(54) **Procédé de préparation de composés halogénés en alpha d'un groupement électro-attracteur.**

(30) Priorité : **12.07.84 FR 8411087**

(43) Date de publication de la demande : **19.02.86 Bulletin 86/08**

(45) Mention de la délivrance du brevet : **30.09.87 Bulletin 87/40**

(84) Etats contractants désignés : **AT BE CH DE FR GB IT LI LU NL SE**

(56) Documents cités : **EP-A- 0 082 781**

(73) Titulaire : **RHONE-POULENC SANTE**
**Les Miroirs 18 Avenue d'Alsace**
**F-92400 Courbevoie Cedex (FR)**

(72) Inventeur : **Mignani, Gérard**
**2 avenue des Frères Lumière**
**F-69008 Lyon (FR)**
Inventeur : **Morel, Didier**
**3 rue Jean Jaurès**
**F-91700 Villiers-sur-Orge (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 172 066 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation de composés halogénés en position α d'un groupement électro-attracteur de formule générale :

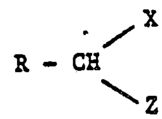

(I)

dans laquelle

X représente un atome d'halogène, de préférence un atome de chlore ou de brome,

R représente un atome d'hydrogène ou un radical hydrocarboné saturé ou un radical hydrocarboné insaturé contenant une ou plusieurs doubles ou triples liaisons, ces radicaux étant éventuellement substitués par un ou plusieurs radicaux, identiques ou différents, choisis parmi les radicaux phényles, alcoyloxycarbonyles, acyles et cyano, ou un radical $-(CH_2)_n$ $COOR_1$ dans lequel n est un nombre entier compris entre 3 et 10 inclusivement, et

Z représente un radical $-CHO$, $-COR_2$, $-COOR_3$, $-CONR_4R_5$, $-CN$, $-SO_2R_6$, $-NO_2$, $-CO-O(CH_2)p-COOR_1$ $-CO-(CH_2)p-COOR_1$, p étant un nombre entier compris entre 2 et 10 inclusivement,

étant entendu que R et Z ne peuvent pas représenter simultanément $-(CH_2)_n-COOR_1$ et $-CO-(CH_2)p-COOR_1$ ou $-CO-O-(CH_2)p-COOR_1$, par déacylation halogénante d'un produit de formule générale :

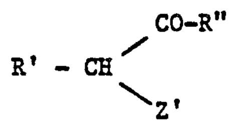

(II)

dans laquelle

R′ représente un atome d'hydrogène ou un radical hydrocarboné saturé ou un radical hydrocarboné insaturé contenant une ou plusieurs doubles ou triples liaisons, ces radicaux étant éventuellement substitués par un ou plusieurs radicaux, identiques ou différents, choisis parmi les radicaux phényles, alcoyloxycarbonyles, acyles et cyano,

R″ représente un radical méthyle

Z′ représente un radical $-CHO$, $-COR_2$, $-COOR_3$, $-CONR_4R_5$, $-CN$, $-SO_2R_6$ et $-NO_2$, étant entendu que R′ et R″ peuvent former ensemble un radical $-(CH_2)_n-$ dans lequel n est défini comme précédemment ou bien que R″ et Z′ peuvent former ensemble un radical $-CO(CH_2)p-$ ou $-CO-O(CH_2)p-$ dans lequel p est défini comme précédemment.

Dans ce qui précède et ce qui suit, les radicaux hydrocarbonés saturés contiennent 1 à 20 atomes de carbone, les radicaux hydrocarbonés insaturés contiennent 2 à 20 atomes de carbone, les radicaux $R_1$, $R_2$, $R_3$, $R_4$, $R_5$, et $R_6$ représentent des radicaux alcoyles contenant 1 à 4 atomes de carbone et les portions alcoyles des radicaux alcoyloxycarbonyles contiennent 1 à 4 atomes de carbone.

Il est connu, en particulier d'après P. L. Stotter et K. A. Hill, Tetrahedron letters, 40, 4067-4070 (1972) de préparer des α-bromoesters par déacylation, au moyen d'hydroxyde de baryum anhydre dans un alcool, d'un bromocétoester obtenu par action du brome sur l'énolate du β-cétoester.

Par ailleurs, il est connu que la déacylation de composés diacétylés peut être effectuée par chauffage en milieu alcoolique basique comme décrit par exemple par S. Boatman et Coll., J. Org. Chem., 30, 3321 (1965) ou par S. Boatman et C. R. Hauser, Organic Syntheses, Coll. Vol. V, 767 (1973).

Il est connu également que le chauffage d'un β-cétoester en présence d'un alcoolate de métal alcalin (méthylate de sodium) dans un alcool (méthanol) conduit à une réaction de déacylation.

Il a maintenant été trouvé, et c'est ce qui fait l'objet de la présente invention, qu'un produit de formule générale (II) traité par un alcoolate de métal alcalin ou par un carbonate de métal alcalin ou alcalino-terreux dans un solvant organique puis par un agent d'halogénation à une température comprise entre $-35$ et $+100$ °C conduit au produit de formule générale (I) par une réaction simultanée de déacylation et d'halogénation.

Pour la mise en œuvre du procédé selon l'invention, il est particulièrement avantageux d'utiliser un alcoolate de métal alcalin dérivé d'un alcool de formule générale $R_1OH$, (III) dans laquelle $R_1$ est défini comme précédemment, tel que le méthylate ou l'éthylate de sodium dans l'alcool correspondant. Afin d'éviter les réactions de transestérification, l'utilisation du méthylate de sodium est particulièrement intéressante. Cependant de bons résultats sont obtenus en utilisant un alcool de formule générale (III) en présence de soude ou de potasse.

Il est possible d'utiliser l'alcoolate de métal alcalin dans un solvant organique anhydre tel que le toluène, l'éther éthylique, le diméthylformamide ou la N-méthylpyrrolidone.

Il est également possible d'utiliser un carbonate de métal alcalin ou alcalino-terreux dans un solvant organique tel que l'acétone ou l'acétonitrile.

Généralement, on utilise une mole d'alcoolate de métal alcalin ou de carbonate alcalin ou alcalino-terreux par mole de produit de formule générale (II) mis en œuvre.

Les agents d'halogénation qui conviennent particulièrement bien sont choisis, de préférence, parmi les halogènes moléculaires (chlore, brome), les N-halogénosuccinimides, le chlorure de sulfuryle et l'hexachloroéthane.

Généralement on utilise une quantité d'agent halogénant légèrement supérieure à la stœchiométrie.

Il est précisé que, selon la présente invention, le produit de formule générale (I) dans laquelle R représente un radical $-(CH_2)_n-COOR_1$ sont obtenus à partir des produits de formule générale (II) dans laquelle R′ et R″ forment ensemble un radical $-(CH_2)_n-$ dans lequel n est un nombre entier compris entre 3 et 10 inclusivement, et que les produits de formule générale (I) dans laquelle Z représente un radical $-CO-(CH_2)p-COOR_1$ ou $-CO-O-(CH_2)p-COOR_1$ sont obtenus à partir des produits de formule générale (II) dans laquelle Z et R″ forment ensemble un radical $-CO-(CH_2)p-$ ou $CO-O-(CH_2)p$ dans lequel p est un nombre entier compris entre 2 et 10 inclusivement, par action d'un alcoolate de métal alcalin dérivé d'un alcool de formule générale (III) et d'un agent d'halogénation.

Il est précisé également que, lorsque dans le produit de formule générale (II), R″ représente un radical méthyle, il se forme, à côté du produit de formule générale (I), un ester de formule générale :

$$CH_3COOR_1 \qquad (IV)$$

dans laquelle $R_1$ est défini comme précédemment.

Les produits de formule générale (I) obtenus selon le procédé de la présente invention peuvent être isolés du mélange réactionnel selon les méthodes habituelles d'extraction et ils peuvent être purifiés par application des méthodes physico-chimiques telle que la distillation ou la chromatographie.

Les produits de formule générale (I) sont particulièrement intéressants comme intermédiaires en chimie organique. Par exemple, les produits de formule générale (I) dans laquelle Z représente un radical $-COR_2$ peuvent conduire facilement à la pseudo-ionone qui est un intermédiaire utilisable dans la synthèse de la vitamine A.

La pseudo-ionone peut être obtenue par déhydrohalogénation du produit de formule :

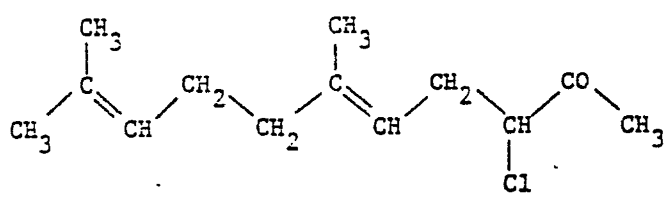

et/ou

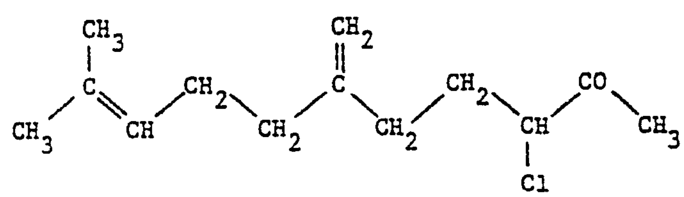

au moyen de chlorure de lithium dans un solvant aprotique polaire basique tel que la N-méthylpyrrolidone à une température comprise entre 80 et 160 °C pendant 1 à 20 heures.

Les exemples suivants, donnés à titre non limitatif, montrent comment l'invention peut être mise en pratique.

## Exemple 1

Dans un ballon tricol de 250 $cm^3$, on introduit, sous atmosphère d'argon, 100 $cm^3$ de méthanol et, par petites fractions, 1,39 g de sodium (60,4 milli-atome-grammes). Après refroidissement à −35 °C, on ajoute 15,24 g (60,4 m · moles) d'un mélange 55/45 de :

$$(CH_3)_2C{=}CH-CH_2CH_2-C({=}CH_2)-CH_2-CH_2-CH\ (COOCH_3)-COCH_3$$

et

$$(CH_3)_2C{=}CH-CH_2CH_2-C(CH_3){=}CH-CH_2-CH(COOCH_3)\ COCH_3$$

puis 8,17 g de N-chlorosuccinimide (61 m · moles). On laisse la température remonter à 25 °C puis on

maintient à cette température pendant 6 heures. Le mélange réactionnel est repris par 100 cm³ d'eau puis il est extrait par 4 fois 50 cm³ d'éther éthylique. Les phases organiques réunies sont séchées sur sulfate de sodium. Après filtration et évaporation du solvant, on obtient 13,65 g d'une huile incolore qui, après distillation sous pression réduite (0,8 mm de mercure ; 0,11 kPa) à 102 °C, fournit 9,31 g d'un mélange 55/45 de :

$(CH_3)_2C{=}CH{-}CH_2CH_2{-}C({=}CH_2){-}CH_2{-}CH_2{-}CHCl{-}COOCH_3$
et
$(CH_3)_2C{=}CH{-}CH_2CH_2{-}C(CH_3){=}CH{-}CH_2{-}CHCl{-}COOCH_3$

Le rendement en produit isolé est de 63,5 %.

Un dosage par chromatographie en phase gazeuse, avec un étalon interne, du produit brut de réaction montre que le taux de transformation de l'ester de départ est de 100 % et que le rendement en composés chlorés est de 72 %.

La structure des produits obtenus est confirmée par le spectre infra-rouge, le spectre de masse et le spectre de résonance magnétique nucléaire du proton.

## Exemple 2

Dans un ballon tricol de 100 cm³, on introduit, sous atmosphère d'argon, 50 cm³ de méthanol anhydre et, par petites fractions, 0,69 g de sodium (30 milli-atome-grammes). Après refroidissement à — 35 °C, on ajoute 6,77 g (28,7 m · moles) d'un mélange 55/45 de :

$(CH_3)_2C{=}CH{-}CH_2CH_2{-}C({=}CH_2)CH_2{-}CH_2CH(COCH_3)_2$
et
$(CH_2)_2C{=}CH{-}CH_2CH_2{-}C(CH_3){=}CH{-}CH_2CH(COCH_3)_2$

puis 4,08 g de N-chlorosuccinimide (30 m · moles). On laisse la température remonter à 25 °C puis on agite le mélange réactionnel pendant 5 heures 30 minutes à cette température. Le mélange réactionnel est repris par 100 cm³ d'eau puis il est extrait par 4 fois 50 cm³ d'éther éthylique. Les phases organiques réunies sont séchées sur sulfate de sodium. Après filtration et évaporation du solvant, on obtient 6,9 g d'une huile jaune qui, après distillation sous pression réduite (0,9 mm de mercure ; 0,12 kPa) à 91-92 °C, fournit 4,19 g d'un mélange 55/45 de :

$(CH_3)_2C{=}CH{-}CH_2CH_2{-}C({=}CH_2){-}CH_2{-}CH_2{-}CHCl{-}COCH_3$
et
$(CH_3)_2C{=}CH{-}CH_2CH_2{-}C(CH_3){=}CH{-}CH_2{-}CHCl{-}COCH_3$

Le rendement en produit isolé est de 64 %.

Un dosage par chromatographie en phase gazeuse, avec un étalon interne, du produit brut de réaction montre que le taux de transformation de la β-dicétone de départ est voisin de 100 % et que le rendement en composés chlorés est de 84 %.

La structure des produits obtenus est confirmée par le spectre infra-rouge, le spectre de masse et le spectre de résonance magnétique nucléaire du proton.

## Exemple 3

Dans un ballon tricol de 100 cm³, on introduit, sous atmosphère d'argon, 15 cm³ de méthanol anhydre et, par petites fractions, 0,206 g (8,9 milli-atome-grammes) de sodium. Après refroidissement à — 35 °C, on ajoute 3,0 g (9 m · moles) d'un mélange 55/45 de :

$(CH_3)_2C{=}CH{-}CH_2CH_2{-}C({=}CH_2)CH_2{-}CH_2{-}CH(SO_2C_6H_5)COCH_3$
et
$(CH_3)_2C{=}CH{-}CH_2CH_2{-}C(CH_3){=}CH{-}CH_2{-}CH(SO_2C_6H_5)COCH_3$

puis 1,22 g de N-chloro succinimide (9,1 m · moles). On laisse la température remonter à 25 °C puis on agite le mélange réactionnel pendant 16 heures à cette température. Le mélange réactionnel est repris par 100 cm³ d'eau et il est extrait par 4 fois 50 cm³ d'éther éthylique. Les phases organiques réunies sont séchées sur sulfate de sodium. Après filtration et évaporation du solvant, on obtient 2,78 g d'une huile jaune dont l'analyse par résonance magnétique nucléaire du proton montre qu'elle contient 90 % d'un mélange 55/45 de :

$(CH_3)_2C{=}CH{-}CH_2CH_2{-}C({=}CH_2)CH_2{-}CH_2CHCl{-}SO_2C_6H_5$
et
$(CH_3)_2C{=}CH{-}CH_2CH_2{-}C(CH_3){=}CH{-}CH_2CHCl{-}SO_2C_6H_5$

Le rendement en produit isolé est de 85 %.

La structure des produits obtenus est confirmée par le spectre infra-rouge, le spectre de masse et le spectre de résonance magnétique nucléaire du proton.

Exemple 4

Dans un ballon tricol de 100 $cm^3$, on introduit, sous atmosphère d'argon, 50 $cm^3$ de méthanol anhydre et, par petites fractions, 0,7 g (30 milli-atome-grammes) de sodium. Après refroidissement à — 35 °C, on ajoute 10,03 g (30 m · moles) d'un mélange 55/45 de :

$(CH_3)_2C{=}CH{-}CH_2CH_2{-}CH({=}CH_2)CH_2{-}CH_2{-}CH\ (SO_2C_6H_5)COCH_3$

et

$(CH_3)_2{=}CH{-}CH_2CH_2{-}C(CH_3){=}CH{-}CH_2{-}CH(SO_2C_6H_5)COCH_3$

puis 5,31 g (30 m · moles) de N-bromosuccinimide. On laisse la température remonter à 25 °C puis on agite le mélange réactionnel pendant 16 heures à cette température. Le mélange réactionnel est repris par 100 $cm^3$ d'eau et il est extrait par 4 fois 50 $cm^3$ d'éther éthylique. Les phases organiques réunies sont séchées sur sulfate de sodium. Après filtration et évaporation du solvant, on obtient 10,55 g d'une huile dont l'analyse par résonance magnétique nucléaire du proton montre qu'elle est constituée d'un mélange 55/45 de :

$(CH_3)_2C{=}CH{-}CH_2CH_2C({=}CH_2)CH_2{-}CH_2{-}CHBr{-}SO_2C_6H_5$

et

$(CH_3)_2C{=}CH{-}CH_2CH_2{-}C(CH_3){=}CH{-}CH_2{-}CHBr{-}SO_2C_6H_5$

Le rendement en produit isolé est de 95 %.

La structure des produits obtenus est confirmée par le spectre infra-rouge, le spectre de masse et le spectre de résonance magnétique nucléaire du proton.

Exemple 5

Dans un ballon tricol de 100 $cm^3$, on introduit, sous atmosphère d'argon, 50 $cm^3$ de méthanol et, par petites fractions, 0,79 g (34,4 milli-atome-grammes) de sodium. Après refroidissement à — 35 °C, on ajoute 7,74 g (33 m · moles) d'un mélange 55/45 de :

$(CH_3)_2C{=}CH{-}CH_2CH_2{-}C({=}CH_2)CH_2{-}CH_2{-}CH(COCH_3)_2$

et

$(CH_3)_2C{=}CH{-}CH_2CH_2{-}C(CH_3){=}CH{-}CH_2CH(COCH_3)_2$

puis 4,41 g (33 m · moles) de N-chlorosuccinimide. On laisse remonter la température à 25 °C puis on agite le mélange réactionnel pendant 16 heures à cette température. Le mélange réactionnel est repris par 100 $cm^3$ d'eau puis il est extrait par 2 fois 50 $cm^3$ d'éther éthylique. Les phases organiques réunies sont séchées sur sulfate de sodium. Après filtration et évaporation du solvant, on obtient 5,3 g (23 m · moles) d'un mélange 55/45 de :

$(CH_3)_2C{=}CHCH_2CH_2{-}C({=}CH_2)CH_2{-}CH_2CHCl{-}COCH_3$

et

$(CH_3)_2C{=}CH{-}CH_2CH_2{-}C(CH_3){=}CH{-}CH_2{-}CHCl{-}COCH_3$

Le rendement en produit isolé est de 70 %.

La β-dicétone de départ, sous forme d'un mélange 55/45, peut être préparée de la manière suivante :

Dans un autoclave en acier inoxydable de 125 $cm^3$, on introduit, sous atmosphère d'argon, 20 $cm^3$ d'un mélange eau-méthanol (75-25 en volumes), 0,302 g de carbonate de sodium, 0,833 g de tri-(m. sulfophényl) phosphine sous forme de sel de sodium, 0,0686 g de $[RhCl\ (cyclooctadiène\text{-}1,5)]_2$ soit 0,0027 milli-atome-gramme de rhodium, 8,01 g d'acétylacétone (80 m · moles) et 16,4 g de myrcène (120,6 m · moles). On laisse réagir pendant 16 heures à 90 °C.

Après refroidissement, on ajoute 40 $cm^3$ d'éther éthylique puis on transfère le mélange réactionnel dans une ampoule à décanter. La phase aqueuse, qui est séparée par décantation, est extraite par 25 $cm^3$ d'éther éthylique. Les phases organiques réunies sont séchées sur sulfate de sodium. Après filtration et évaporation du solvant, on obtient 21,73 g d'une huile orangée qui contient 15,21 g d'un mélange 55/45 de :

$(CH_3)_2C{=}CH{-}CH_2CH_3{-}C({=}CH_2)CH_2{-}CH_2{-}CH(COCH_3)_2$

et

$(CH_3)_2C{=}CH{-}CH_2CH_2{-}C(CH_3){=}CH{-}CH_2{-}CH(COCH_3)_2$

le rendement en produit isolé est de 80,6 %.

Exemple 6

Dans un ballon tricol de 100 $cm^3$, on introduit, sous atmosphère d'argon, 30 $cm^3$ de méthanol anhydre et, par petites fractions, 0,69 g de sodium (30 milli-atome-grammes). Après refroidissement à — 35 °C, on ajoute 5,16 g (30 m · moles) de :

$$CH_3(CH_2)_3—CH(COCH_3)COOCH_3$$

puis 4,0 g de N-chlorosuccinimide (30 m · moles). On laisse la température remonter à 25 °C puis on agite le mélange réactionnel pendant 18 heures à cette température. Le mélange réactionnel est repris par 100 $cm^3$ d'eau et 100 $cm^3$ d'éther éthylique. La phase aqueuse, séparée par décantation, est extraite par 3 fois 50 $cm^3$ d'éther. Les phases organiques réunies sont séchées sur sulfate de sodium. Après filtration et évaporation du solvant, on obtient une huile incolore qui après une flash-distillation à 100 °C sous une pression de 1 mm de mercure (0,13 kPa) fournit 3,2 g d'une huile incolore contenant 95 % de :

$$CH_3(CH_2)_3CHCl—COOCH_3$$

Le rendement en produit isolé est de 62 %.

La structure du produit obtenue est confirmée par le spectre infra-rouge, le spectre de masse et le spectre de résonance magnétique nucléaire du proton.

Exemple 7

Dans un ballon tricol de 100 $cm^3$ on introduit, sous atmosphère d'argon, 30 $cm^3$ d'éthanol anhydre et par petites fractions, 0,69 g (30 milli-atome-grammes) de sodium. Après refroidissement à — 30 °C, on ajoute 5,1 g d'éthoxycarbonyl-2 cyclohexanone (30 m · moles) et 4,0 g de N-chlorosuccinimide (30 m · moles). On laisse la température remonter à 25 °C puis on agite le mélange réactionnel pendant 10 heures à cette température. Le mélange réactionnel est repris par 100 $cm^3$ d'eau et 100 $cm^3$ d'éther éthylique. Après décantation, la phase aqueuse est extraite par 3 fois 30 $cm^3$ d'éther éthylique. Les phases organiques réunies sont séchées sur sulfate de sodium. Après filtration et évaporation du solvant, on obtient 7,7 g d'une huile jaune qui, après une flash distillation à 200 °C sous une pression de 1 mm de mercure (0,13 kPa) fournit 3,6 g d'une huile incolore contenant 90 % de diéthoxycarbonyl-1,5 chloro-1 pentane.

La structure du produit obtenu est confirmée par le spectre infra-rouge, le spectre de masse et le spectre de résonance magnétique nucléaire du proton.

Exemple 8

Dans un ballon tricol de 100 $cm^3$, on introduit, sous atmosphère d'argon, 30 $cm^3$ de méthanol et, par petites fractions, 0,69 g de sodium (30 milli-atome-grammes). Après refroidissement à — 30 °C, on ajoute 5,52 g (30 m · moles) d'un mélange 60/40 de :

$$CH_2{=}C(CH_3)CH_2CH_2CH(COCH_3)COOCH_3$$

et

$$(CH_3)_2C{=}CH—CH_2CH(COCH_3)COOCH_3$$

et 4,0 g de N-chlorosuccinimide (30 m · moles). On laisse la température remonter à 25 °C puis on agite le mélange réactionnel pendant 10 heures à cette température. Le mélange réactionnel est repris par 100 $m^3$ d'eau et 100 $cm^3$ d'éther éthylique. Après décantation, la phase aqueuse est extraite par 3 fois 30 $cm^3$ d'éther éthylique. Les phases organiques réunies sont séchées sur sulfate de sodium. Après filtration et évaporation du solvant, on obtient 5,7 g d'une huile jaune qui, après une flash distillation à 150 °C sous une pression de 20 mm de mercure (2,6 kPa) fournit 3,5 g d'une huile incolore contenant un mélange 60/40 de :

$$CH_2{=}C(—CH_3)—CH_2CH_2—CHCl—COOCH_3$$

et

$$(CH_3)_2C{=}CH—CH_2—CHCl—COOCH_3$$

Le rendement en produit isolé est de 66 %.

La structure des produits obtenus est confirmée par le spectre infra-rouge, le spectre de masse et le spectre de résonance magnétique nucléaire du proton.

Exemple 9

Dans un ballon tricol de 100 $cm^3$, on introduit, sous atmosphère d'argon, 30 $cm^3$ de méthanol et, par petites fractions, 0,46 g (20 milli-atome-grammes) de sodium. Après refroidissement à — 30 °C on ajoute 5,04 g (30 m · moles) d'un mélange 55/45 de :

$$(CH_3)_2C{=}CH{-}CH_2CH_2{-}C({=}CH_2)CH_2CH_2CH(COOCH_3)COCH_3$$
et
$$(CH_3)_2C{=}CH{-}CH_2CH_2{-}C(CH_3){=}CH{-}CH_2CH(COOCH_3)COCH_3$$

puis 3,56 g de N-bromosuccinimide (20 m · moles). On laisse la température remonter à 25 °C puis on agite le mélange réactionnel pendant 10 heures à cette température. En fin de réaction, le mélange réactionnel est homogène et jaune clair. Le mélange réactionnel est repris par 100 $cm^3$ d'eau et 100 $cm^3$ d'éther éthylique. Après décantation, la phase aqueuse est extraite par 3 fois 30 $cm^3$ d'éther. Les phases organiques réunies sont séchées sur sulfate de sodium. Après filtration et évaporation du solvant, on obtient 5,1 g d'une huile jaune qui, après flash distillation à 200 °C sous une pression de 1 mm de mercure (0,13 kPa), fournit 3,5 g d'une huile incolore contenant 85 % d'un mélange 55/45 de :

$$(CH_3)_2C{=}CH{-}CH_2CH_2{-}C({=}CH_2)CH_2{-}CH_2{-}CHBr{-}COOCH_3$$
et
$$(CH_3)_2C{=}CH{-}CH_2CH_2{-}C(CH_3){=}CH{-}CH_2CHBr{-}COOCH_3$$

La structure des produits obtenus est confirmée par les spectres infra-rouge, le spectre de masse et le spectre de résonance magnétique nucléaire du proton.

Exemple 10

Dans un ballon tricol de 100 $cm^3$, on introduit, sous atmosphère d'argon, 30 $cm^3$ de méthanol anhydre et, par petites fractions, 0,69 g de sodium (30 milli-atome-grammes). Après refroidissement à — 20 °C, on ajoute 5,52 g (30 m · moles) d'un mélange 60/40 de :

$$CH_2{=}C(CH_3){-}CH_2CH_2{-}CH(COCH_3)COOCH_3$$
et
$$(CH_3)_2C{=}CH{-}CH_2CH_2{-}CH(COCH_3)COOCH_3$$

et 5,33 g de N-bromosuccinimide (30 m · moles). On laisse remonter la température à 25 °C puis on agite le mélange réactionnel pendant 10 heures à cette température. Le mélange réactionnel est repris par 100 $cm^3$ d'eau et 100 $cm^3$ d'éther anhydre. Après décantation, la phase aqueuse est extraite par 3 fois 30 $cm^3$ d'éther éthylique. Les phases organiques réunies sont séchées sur sulfate de sodium. Après filtration et évaporation du solvant, on obtient 6,1 g d'une huile jaune qui, après une flash distillation, fournit 3,9 g d'une huile incolore.

L'analyse par résonance magnétique nucléaire du proton montre que le produit brut de la réaction est constitué de 85 % d'un mélange de

$$CH_2{=}C(CH_3)CH_2CH_2{-}CHBr{-}COOCH_3$$
et
$$(CH_3)_2C{=}CH{-}CH_2CH_2{-}CHBr{-}COOCH_3$$

et de 15 % de β-cétoesters de départ.

La structure des produits obtenus est confirmée par le spectre infra-rouge, le spectre de masse, et le spectre de résonance magnétique nucléaire du proton.

Exemple 11

Dans un ballon tricol de 100 $cm^3$, on introduit, sous atmosphère d'argon, 25 $cm^3$ de méthanol anhydre et, par petites fractions, 0,45 g (19,5 milli-atome-grammes) de sodium. On ajoute ensuite 5,92 g (23,5 m · moles) d'un mélange 55/45 de :

$$(CH_3)_2C{=}CH{-}CH_2CH_2C({=}CH_2)CH_2CH_2{-}CH(COCH_3)COOCH_3$$
et
$$(CH_3)_2C{=}CH{-}CH_2CH_2{-}C(CH_3){=}CH{-}CH_2CH(COCH_3)COOCH_3,$$

puis on refroidit à une température comprise entre 5 et 8 °C. On ajoute une solution de 1,7 g (24 m · moles) de chlore dans 20 $cm^3$ de tétrachlorure de carbone. On agite le mélange réactionnel pendant 5

heures à 5 °C. Le mélange réactionnel est repris par 100 $cm^3$ d'eau et 100 $cm^3$ d'éther éthylique. Après décantation, la phase aqueuse est extraite par l'éther éthylique. Les phases organiques réunies sont séchées sur sulfate de sodium. Après filtration et évaporation du solvant, on obtient un produit brut de réaction qui contient 30 % d'un mélange 55/45 de :

$$(CH_3)_2C{=}CH{-}CH_2CH_2{-}C({=}CH_2){-}CH_2CH_2{-}CHCl{-}COOCH_3$$
et
$$(CH_3)_2C{=}CH{-}CH_2CH_2{-}C(CH_3){=}CH_2{-}CH_2CHCl{-}COOCH_3$$

## Exemple 12

Dans un ballon tricol de 100 $cm^3$, on introduit, sous atmosphère d'argon, 20 $cm^3$ de méthanol anhydre et, par petites fractions, 0,23 g (10 milli-atome-grammes) de sodium, puis on ajoute 2,52 g (10 m · moles) d'un mélange 55/45 de :

$$(CH_3)_2C{=}CH{-}CH_2CH_2{-}C({=}CH_2)CH_2CH_2CH(COCH_3)COOCH_3$$
et
$$(CH_3)_2C{=}CH{-}CH_2CH_2{-}C(CH_3){=}CHCH_2CH(COCH_3)COOCH_3,$$

et lentement, à une température voisine de 20 °C, 0,8 $cm^3$ (10 m · moles) de chlorure de sulfuryle. On laisse réagir pendant 6 heures. Le mélange réactionnel est repris par 100 $cm^3$ d'eau et 100 $cm^3$ d'éther éthylique. Après décantation, la phase aqueuse est extraite par 3 fois 30 $cm^3$ d'éther éthylique. Les phases organiques réunies sont séchées sur sulfate de sodium. Après filtration et évaporation du solvant, on obtient 2,6 g d'une huile colorée.

L'analyse par chromatographie en phase vapeur montre que le produit brut de la réaction contient 45 % des β-cétoesters de départ et 50 % d'un mélange de :

$$(CH_3)_2C{=}CH{-}CH_2CH_2{-}C({=}CH_2)CH_2CH_2{-}CHCl{-}COOCH_3$$
et
$$(CH_3)_2C{=}CH{-}CH_2CH_2{-}C(CH_3){=}CH{-}CH_2{-}CHCl{-}COOCH_3$$

## Exemple 13

Dans un ballon de 100 $cm^3$, on introduit, sous atmosphère d'argon, 20 $cm^3$ de méthanol, 0,56 g (0,02 mole) de potasse broyée et 2,52 g (0,01 mole) d'un mélange 55/45 de :

$$(CH_3)_2C{=}CH{-}CH_2CH_2{-}C({=}CH_2){-}CH_2CH_2{-}CH(COOCH_3)COCH_3$$
et
$$(CH_3)_2C{=}CH{-}CH_2CH_2{-}C(CH_3){=}CH{-}CH_2{-}CH(COOCH_3)COCH_3$$

On ajoute ensuite 1,4 g (0,0104 mole) de N-chlorosuccinimide puis on laisse réagir pendant 2 heures à 25 °C. La solution devient homogène. Le mélange réactionnel est repris par 100 $cm^3$ d'eau puis on extrait par 3 fois 50 $cm^3$ d'éther. Les phases organiques réunies, et séchées sur sulfate de magnésium. Après filtration et évaporation du solvant, on obtient une huile légèrement jaune qui contient 70 % d'un mélange 55/45 de :

$$(CH_3)_2C{=}CH{-}CH_2CH_2{-}C({=}CH_2){-}CH_2CH_2{-}CHCl{-}COOCH_3$$
et
$$(CH_3)_2C{=}CH{-}CH_2CH_2{-}C(CH_3){=}CH{-}CH_2{-}CHCl{-}COOCH_3$$

Après une flash distillation, on obtient 2,0 g du mélange des composés chlorés ayant une pureté de 93 %.

Le rendement en produit isolé est de 76 %.

## Exemple 14 : Préparation de la pseudo-ionone.

A 0,5 g de chlorure de lithium (11,7 m · moles) dans 20 $cm^3$ de N-méthylpyrrolidone, on ajoute 1,29 g de triméthyl-2,4,6 pyridine (10,6 m · moles) et 1,94 g du mélange des produits obtenus à l'exemple 5.

Le mélange est chauffé à 150 °C pendant 2 heures 30 minues. Après traitement du mélange réactionnel on obtient, avec un rendement de 88 %, 1,44 g de pseudo-ionone.

La sélectivité en pseudo-ionone est de 94,6 % pour un taux de déhydrochloration de 93 %.

## Exemple 15

Dans un ballon de 100 $cm^3$, on introduit, sous atmosphère d'argon, 20 $cm^3$ d'acétone, 2,76 g (0,02

mole) de carbonate de potassium, 4,73 g (0,02 mole) d'hexachloroéthane et 2,53 g (0,01 mole) d'un mélange 55/45 de :

$$(CH_3)_2C{=}CH{-}CH_2CH_2{-}C({=}CH_2)CH_2CH_2{-}CH(COCH_3){-}COOCH_3$$
et
$$(CH_3)_2C{=}CH{-}CH_2CH_2{-}C(CH_3){=}CH{-}CH_2{-}CH(COCH_3){-}COOCH_3$$

On chauffe le mélange réactionnel hétérogène à reflux pendant 75 heures. Après refroidissement, le mélange réactionnel est filtré puis est repris par 100 $cm^3$ d'eau. Après extraction par 3 fois 50 $cm^3$ de pentane, les couches organiques réunies sont séchées sur sulfate de sodium. Après filtrtion, évaporation du solvant et flash chromatographie, on isole 1,86 g d'un mélange 55/45 de :

$$(CH_3)_2C{=}CH{-}CH_2CH_2{-}C({=}CH_2)CH_2CH_2{-}CCl{-}COOCH_3$$
et
$$(CH_3)_2C{=}CH{-}CH_2CH_2{-}C(CH_3){=}CH{-}CH_2{-}CCl{-}COOCH_3$$

Le rendement en produit isolé est de 76 %.

## Exemple 16

Dans un ballon de 100 $cm^3$, on introduit, sous atmosphère d'argon, 40 $cm^3$ d'éthanol anhydre, 0,45 g de sodium. La réaction étant terminée, on ajoute 4,3 g (19,5 m · moles) de phényl-3 acétyl-2 propionate d'éthyle [$C_6H_5{-}CH_2{-}CH(COCH_3){-}COOC_2H_5$] et 2,7 g (20,2 m · moles) de N-chlorosuccinimide. On laisse réagir pendant 3 heures à une température voisine de 20 °C. le mélange réactionnel est versé dans 100 $cm^3$ d'eau puis on extrait par 3 fois 50 $cm^3$ d'hexane. Les phases organiques réunies sont séchées sur sulfate de sodium. Après filtration et évaporation du solvant, on obtient 3,66 g d'une huile jaune pâle, dont l'analyse par chromatographie en phase gazeuse montre qu'elle contient 98 % de phényl-3 chloro-2 propionate d'éthyle [$C_6H_5{-}CH_2CHCl{-}COOC_2H_5$].

Le rendement est de 86 %.

La structure du produit obtenu est confirmée par le spectre infra-rouge, le spectre de résonance magnétique nucléaire du proton et le spectre de masse.

Le phényl-3 chloro-2 propionate d'éthyle ainsi obtenu peut être transformé en phénylalanine selon les méthodes connues.

## Exemple 17

Dans un ballon de 100 $cm^3$, on introduit, sous atmosphère d'argon, 50 $cm^3$ d'éthanol anhydre et 0,18 g de lithium. On laisse réagir puis on ajoute 4,2 g (19 m · moles) de phényl-3 acétyl-2 propionate d'éthyle puis 4,6 g (25,8 m · moles) de N-bromosuccinimide. On laisse réagir pendant 1 heure à une température voisine de 20 °C. Le mélange réactionnel est repris par 100 $cm^3$ d'eau et le pH est amené à 1. On extrait alors par 3 fois 50 $cm^3$ d'hexane. Les phases organiques réunies sont séchées sur sulfate de sodium. Après filtration et évaporation du solvant, on obtient 4,91 g d'une huile légèrement jaune dont l'analyse par chromatographie en phase gazeuse montre qu'elle contient 93 % de phényl-3 bromo-2 propionate d'éthyle.

le rendement est de 93 %.

La structure du produit obtenu est confirmée par le spectre infra-rouge, le spectre de résonance magnétique nucléaire du proton et le spectre de masse.

## Exemple 18

Dans un ballon de 100 $cm^3$, on introduit, sous atmosphère d'argon, 50 $cm^3$ d'éthanol anhydre et 0,26 g de lithium. On laisse réagir puis on ajoute 4,13 g (24 m · moles) de méthyl-3 acétyl-2 butanoate d'éthyle [$(CH_3)_2CH{-}CH(COCH_3)COOC_2H_5$] et 4,98 g (37,3 m.moles) de N-chlorosuccinimide. On laisse réagir pendant 3 heures à une température voisine de 20 °C. Le mélange réactionnel est repris par 100 $cm^3$ d'eau et le pH est amené à 1. On extrait alors par 3 fois 50 $cm^3$ d'hexane. Les phases organiques réunies sont séchées sur sulfate de sodium. Après filtration et évaporation du solvant on obtient 3,7 g d'une huile légèrement jaune dont l'analyse par chromatographie en phase gazeuse montre qu'elle contient 82 % de méthyl-3 chloro-2 butanoate d'éthyle [$(CH_3)_2CH{-}CHCl{-}COOC_2H_5$].

Le rendement est de 77 %.

La structure du produit obtenu est confirmée par le spectre infra-rouge, le spectre de résonance magnétique nucléaire du proton et le spectre de masse.

**Revendications**

1. Procédé de préparation de composés halogénés en position α d'un groupement électro-attracteur de formule générale :

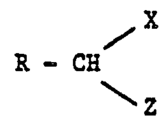

(I)

dans laquelle

X représente un atome d'halogène,

R représente un atome d'hydrogène ou un radical hydrocarboné saturé ou un radical hydrocarboné insaturé contenant une ou plusieurs doubles ou triples liaisons, ces radicaux étant éventuellement substitués par un ou plusieurs radicaux, identiques ou différents, choisis parmi les radicaux phényles, alcoyloxycarbonyles, acyles et cyano, ou un radical $-(CH_2)_n-COOR_1$ dans lequel n est un nombre entier compris entre 3 et 10 inclusivement, et

Z représente un radical $-CHO$, $-COR_2$, $-COOR_3$, $-CONR_4R_5$, $-CN$, $-SO_2R_6$, $-NO_2$, $-CO-(CH_2)p-COOR_1$ ou $-CO-O(CH_2)_p-COOR_1$, p étant un nombre entier compris entre 2 et 10 inclusivement, étant entendu que R et Z ne peuvent pas représenter simultanément $-(CH_2)_n-COOR_4$ et $-CO(CH_2)p-COOR_1$ ou $-CO-O(CH_2)p-COOR_1$, par déacylation halogénante d'un produit de formule générale :

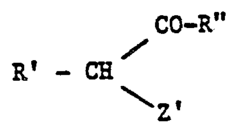

(II)

dans laquelle :

R′ représente un atome d'hydrogène ou un radical hydrocarboné saturé ou un radical hydrocarboné insaturé contenant une ou plusieurs doubles ou triples liaisons, ces radicaux étant éventuellement substitués par un ou plusieurs radicaux identiques ou différents choisis parmi les radicaux phényles, alcoyloxycarbonyles, acyles et cyano,

R″ représente un radical méthyle

Z′ représente un radical $-CHO$, $-COR_2$, $-COOR_3$, $-CONR_4R_5$, $-CN$, $-SO_2R_6$ et $NO_2$,

étant entendu que R′ et R″ peuvent former ensemble un radical $-(CH_2)_n-$ dans lequel n est défini comme précédemment ou bien que R″ et Z′ peuvent former ensemble un radical $-CO-(CH_2)_p-$ ou $-COO(CH_2)_p-$ dans lequel p est défini comme précédemment,

et étant entendu que les radicaux hydrocarbonés saturés contiennent 1 à 20 atomes de carbone, les radicaux hydrocarbonés insaturés contiennent 2 à 20 atomes de carbone, les radicaux $R_1$, $R_2$, $R_3$, $R_4$, $R_5$ et $R_6$ contiennent 1 à 4 atomes de carbone et les portions alcoyles des radicaux alcoyloxycarbonyles contiennent 1 à 4 atomes de carbone,

au moyen d'un alcoolate de métal alcalin ou d'un carbonate alcalin ou alcalino-terreux dans un solvant organique et d'un agent d'halogénation à une température comprise entre −35 et 100 °C.

2. Procédé selon la revendication 1 caractérisé en ce que l'on utilise un alcoolate de métal alcalin dérivé d'un alcool de formule générale :

$$R_1-OH$$

dans laquelle $R_1$ représente un radical alcoyle contenant 1 à 4 atomes de carbone, dans un solvant organique.

3. Procédé selon la revendication 2 caractérisé en ce que l'alcoolate de métal alcalin est choisi parmi le méthylate de sodium et l'éthylate de sodium.

4. Procédé selon la revendication 1 caractérisé en ce que l'on utilise un alcool de formule générale :

$$R_1OH$$

dans laquelle $R_1$ représente un radical alcoyle contenant 1 à 4 atomes de carbone en présence de soude ou de potasse.

5. Procédé selon la revendication 2 caractérisé en ce que le solvant organique est choisi parmi l'alcool de formule générale $R_1OH$, le toluène, l'éther éthylique, le diméthylformamide ou la N-méthylpyrrolidone.

6. Procédé selon la revendication 1 caractérisé en ce que l'on utilise un carbonate de métal alcalin ou alcalino-terreux dans un solvant organique choisi parmi l'acétone et l'acétonitrile.

7. Procédé selon la revendication 1 caractérisé en ce que l'agent d'halogénation est choisi parmi les halogènes moléculaires, les N-halogénosuccinimides, le chlorure de sulfuryle et l'hexachloroéthane.

**Claims**

1. Process for the preparation of compounds halogenated in the α position with respect to an electron-withdrawing group of general formula :

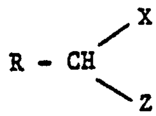

(I)

in which

X denotes a halogen atom,

R denotes a hydrogen atom or a saturated hydrocarbon radical or an unsaturated hydrocarbon radical containing one or more double or triple bonds, it being possible for these radicals to be optionally substituted by one or more identical or different radicals chosen from phenyl, alkoxycarbonyl, acyl and cyano radicals, or a $-(CH_2)_n-COOR_1$ radical in which n is an integer from 3 to 10 inclusive, and

Z denotes a $-CHO$, $-COR_2$, $-COOR_3$, $-CONR_4R_5$, $-CN$, $-SO_2R_6$, $-NO_2$, $-CO-(CH_2)_p-COOR_1$ or $-CO-O(CH_2)_p-COOR_1$ radical, p being an integer from 2 to 10 inclusive, it being understood that R and Z cannot simultaneously denote $-(CH_2)_n-COOR_4$ and $-CO(CH_2)_p-COOR_1$ or $-CO-O(CH_2)_p-COOR_1$, by halogenating deacylation of a product of general formula :

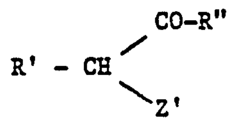

(II)

in which :

R' denotes a hydrogen atom or a saturated hydrocarbon radical or an unsaturated hydrocarbon radical containing one or more double or triple bonds, it being possible for these radicals to be optionally substituted by one or more identical or different radicals chosen from phenyl, alkoxycarbonyl, acyl and cyano radicals,

R" denotes a methyl radical

Z' denotes a $-CHO$, $-COR_2$, $-COOR_3$, $-CONR_4R_5$, $-CN$, $-SO_2R_6$ and $NO_2$ radical,

it being understood that R' and R" may together form a $-(CH_2)_n-$ radical in which n is defined as above or alternatively that R" and Z' may together form a $-CO-(CH_2)_p-$ or $-COO(CH_2)_p-$ radical in which p is defined as above, and

it being understood that the saturated hydrocarbon radicals contain 1 to 20 carbon atoms, the unsaturated hydrocarbon radicals contain 2 to 20 carbon atoms, the radicals $R_1$, $R_2$, $R_3$, $R_4$, $R_5$ and $R_6$ contain 1 to 4 carbon atoms and the alkyl moieties of the alkoxycarbonyl radicals contain 1 to 4 carbon atoms,

by means of an alkali metal alcoholate or an alkali metal or alkaline-earth metal carbonate in an organic solvent and of a halogenating agent at a temperature of between −35 and 100 °C.

2. Process according to Claim 1, characterized in that an alkali metal alcoholate derived from an alcohol of general formula

$$R_1-OH$$

in which $R_1$ denotes an alkyl radical containing 1 to 4 carbon atoms, is used in an organic solvent.

3. Process according to Claim 2, characterized in that the alkali metal alcoholate is chosen from sodium methylate and sodium ethylate.

4. Process according to Claim, characterized in that an alcohol of general formula :

$$R_1OH$$

in which $R_1$ denotes an alkyl radical containing 1 to 4 carbon atoms, is used in the presence of sodium hydroxide or potassium hydroxide.

5. Process according to Claim 2, characterized in that the organic solvent is chosen from the alcohol of general formula $R_1OH$, toluene, ethyl ether, dimethylformamide or N-methylpyrrolidone.

6. Process according to Claim 1, characterized in that an alkali metal or alkaline-earth metal carbonate is used in an organic solvent chosen from acetone and acetonitrile.

7. Process according to Claim 1, characterized in that the halogenating agent is chosen from molecular halogens, N-halosuccinimides, sulphuryl chloride and hexachloroethane.

**Patentansprüche**

1. Verfahren zur Herstellung von in α-Stellung zu einer elektronen-anziehenden Gruppe halogenierten Verbindungen der allgemeinen Formel :

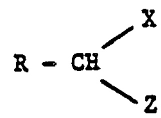

(I)

in welcher

X ein Halogenatom darstellt,

R ein Wasserstoffatom oder einen gesättigten Kohlenwasserstoffrest oder einen ungesättigten Kohlenwasserstoffrest mit einer oder mehreren Doppel- oder Dreifachbindungen darstellt, welche Reste gegebenenfalls durch einen oder mehrere gleiche oder voneinander verschiedene Reste, ausgewählt aus den Phenyl-, Alkyloxycarbonyl-, Acyl- und Cyanoresten, oder einen Rest $-(CH_2)_n-COOR_1$, worin n eine ganze Zahl zwischen 3 und 10 inklusive ist, substituiert sein können, und

Z einen Rest $-CHO$, $-COR_2$, $-COOR_3$, $-CONR_4R_5$, $-CN$, $-SO_2R_6$, $-NO_2$, $-CO-(CH_2)_p-COOR_1$ oder $-CO-O(CH_2)_p-COOR_1$, darstellt, worin p eine ganze Zahl zwischen 2 und 10 inklusive ist und R und Z selbstverständlich nicht gleichzeitig $-(CH_2)_n-COOR_4$ und $-CO(CH_2)_p-COOR_1$ oder $-CO-O(CH_2)_p-COOR_1$ darstellen können, durch halogenierende Deacylierung einer Verbindung der allgemeinien Formel :

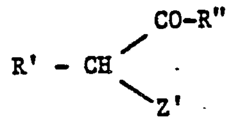

(II)

in welcher

R' ein Wasserstoffatom oder einen gesättigten Kohlenwasserstoffrest oder einen ungesättigten Kohlenwasserstoffrest mit einer oder mehreren Doppel- oder Dreifachbindungen darstellt, welche Reste gegebenenfalls durch einen oder mehrere gleiche oder voneinander verschiedene Reste, ausgewählt aus den Phenyl-, Alkyloxycarbonyl-, Acyl- und Cyanoresten, substituiert sind,

R" einen Methylrest darstellt,

Z' einen Rest $-CHO$, $-COR_2$, $-COOR_3$, $-CONR_4R_5$, $-CN$, $-SO_2R_6$ und $-NO_2$ darstellt, wobei selbstverständlich R' und R" zusammen einen Rest $-(CH_2)_n-$ darstellen können, worin n die obige Bedeutung hat, oder R" und Z' zusammen einen Rest $-CO-(CH_2)_p-$ oder $-COO(CH_2)_p-$ darstellen können, worin p die obige Bedeutung hat,

und wobei selbstverständlich die gesättigten Kohlenwasserstoffreste 1 bis 20 Kohlenstoffatome enthalten, die ungesättigten Kohlenwasserstoffreste 2 bis 20 Kohlenstoffatome enthalten, die Reste $R_1$, $R_2$, $R_3$, $R_4$, $R_5$ und $R_6$ 1 bis 4 Kohlenstoffatome enthalten und die Alkylteile der Alkyloxycarbonylreste 1 bis 4 Kohlenstoffatome enthalten,

mittels eines Alkalimetallalkoholats oder eines Alkali- oder Erdalkalicarbonats in einem organischen Lösungsmittel und einem Halogenierungsmittel bei einer Temperatur zwischen $-35$ und 100 °C.

2. Verfahren nach Anspruch 1, dadurch gekennzeichnet, daß man ein von einem Alkohol der allgemeinen Formel

$$R_1-OH$$

in welcher $R_1$ einen Alkylrest mit 1 bis 4 Kohlenstoffatomen darstellt, abgeleitetes Alkalimetallalkoholat in einem organischen Lösungsmittel verwendet.

3. Verfahren nach Anspruch 2, dadurch gekennzeichnet, daß das Alkalimetallalkoholat ausgewählt ist aus Natriummethylat und Natriumäthylat.

4. Verfahren nach Anspruch 1, dadurch gekennzeichnet, daß man einen Alkohol der allgemeinen Formel :

$$R_1OH$$

in welcher $R_1$ einen Alkylrest mit 1 bis 4 Kohlenstoffatomen darstellt, in Gegenwart von Natrium- oder Kaliumhydroxid verwendet.

5. Verfahren nach Anspruch 2, dadurch gekennzeichnet, daß das organische Lösungsmittel ausgewählt ist aus dem Alkohol der allgemeinen Formel $R_1OH$, Toluol, Äthyläther, Dimethylformamid oder N-Methylpyrrolidon.

6. Verfahren nach Anspruch 1, dadurch gekennzeichnet, daß man ein Alkali- oder Erdalkalimetallcarbonat in einem organischen Lösungsmittel, ausgewählt aus Aceton und Acetonitril, verwendet.

7. Verfahren nach Anspruch 1, dadurch gekennzeichnet, das das Halogenierungsmittel ausgewählt wird aus den molekularen Halogenen, den N-Halogensuccinimiden, Sulfurylchlorid und Hexachloräthan.